(19) Europäisches Patentamt

European Patent Office

Office européen des brevets

(11) Publication number : **0 533 416 A1**

# (12) EUROPEAN PATENT APPLICATION

(21) Application number : 92308325.7

(22) Date of filing : 14.09.92

(51) Int. Cl.⁵ : **A61K 37/02**

(30) Priority : **16.09.91 US 760293**

(43) Date of publication of application :
**24.03.93 Bulletin 93/12**

(84) Designated Contracting States :
**PT**

(71) Applicant : **SCHERING CORPORATION**
**2000 Galloping Hill Road**
**Kenilworth New Jersey 07033 (US)**

(72) Inventor : **Chou, Chuan-Chu**
**909 Prospect Street**
**Westfield, New Jersey 07090 (US)**
Inventor : **Meador, Vincent P.**
**5721 Sugar Hills Drive**
**Greenfield, Indiana 46140 (US)**
Inventor : **Plunkett, Marian L.**
**Padre Gardens, Apt. 109, 8148 Genesee**
**Avenue**
**San Diego, California 92122 (US)**

(74) Representative : **Ritter, Stephen David et al**
**Mathys & Squire 10 Fleet Street**
**London EC4Y 1AY (GB)**

(54) **Use of IL-5 to treat solid tumors.**

(57)     Methods are provided for treating solid tumors lacking IL-5 receptors in a mammal by the administration of an effective amount of IL-5.

EP 0 533 416 A1

## BACKGROUND OF THE INVENTION

Interleukin-5 (IL-5) is a lymphokine secreted by activated T cells which is biologically active on B cells and eosinophils. Because IL-5 replaces T lymphocytes in *in vitro* antibody responses to thymus-dependent antigens, it was formerly called T cell replacing factor [TRF; Dutton *et al.*, Prog. Immunol. *1*:355 (1971); Schimpl *et al.*, Nature *237*:15 (1972)]. Because it also stimulates differentiation of B lymphocytes into IgM and IgG plaque-forming cells and the growth of B cell lymphomas *in vitro*, it has also been called B cell growth factor II [BCGFII; Takatsu *et al.*, J Immunol. *124*:2414 (1980)]. More recently, human IL-5 has been shown to induce eosinophil proliferation and differentiation [Lopez *et al.*, J. Exp. Med. *163*:1085 (1986)].

Murine IL-5 consists of 133 amino acid residues, including a signal sequence of 21 residues and three N-glycosylation sites. Deglycosylation does not affect the biological activity of murine IL-5 in a B cell proliferation assay [Tavernier *et al.,* DNA *8*:491 (1989)]. Human IL-5 consists of 134 amino acid residues, including a signal sequence of 22 residues and two N-glycosylation sites. The structures of both proteins have been described by Yokota *et al.* [Proc. Natl. Acad. Sci. USA *84*:7388 (1987)] and Kinashi *et al.* [Nature *324*:70 (1986)]. The degrees of homology of murine and human IL-5 at the nucleotide and amino acid sequence level are 77 and 70%, respectively.

Both murine and human IL-5 exist as a dimer linked by a disulfide bond. Therefore, recombinant human IL-5 migrates in SDS polyacrylamide gel electrophoresis with an apparent molecular weight of 40,000 daltons under non-reducing conditions, and 20-22,000 daltons under reducing conditions [Tsujimoto *et al.*, J. Biochem. *106*:23 (1989)].

The cloning and expression of murine IL-5 has been described, e.g., by Kinashi *et al.* [Nature *324*:70 (1986)] and Takatsu *et al.* [J. Immunol. *134*:382 (1985)]. Human IL-5 cDNA has been isolated using murine IL-5 cDNA as a probe by Azuma *et al.* [Nucleic Acids Res. *14*:9149 (1986)].

Recombinant human IL-5 has been reported to enhance cellular toxicity toward certain unspecified antibody-coated tumor cells and helminthic infestations [Lopez *et al.*, J. Exp. Med. *167*:219 (1988)]. This anti-tumor effect was attributed to the selective stimulation of eosinophils by the IL-5. Other reports, however, suggest that eosinophils may not have anti-tumor effects, and that IL-5 can actually be tumorigenic.

Increased levels of eosinophils (eosinophilia) are associated with some tumors. For example, some cases of carcinoma of the lung are associated with eosinophilia [Spry *et al.*, Heart Vessels *1*:162 (1985); Kodama *et al.*, Cancer (Phila.) *54*: 2313 (1984)], and the extracted tumors can be shown to produce a substance that preferentially stimulates eosinophils *in vitro* (Kodama *et al., supra*). Lopez *et al.* [J. Exp. Med. *167*:219 (1988)] suggest that this substance may be IL-5. Moreover, Blankenstein *et al.* [Eur. J. Immunol. *20*:2699 (1990)] have reported that the transfer of rodent IL-5 *c*DNA or genes into IL-5 -dependent hematopoietic cell lines resulted in both autocrine growth and tumorigenicity in nude mice.

International Patent Application Publication No. WO 88/06891 describes the use of a variety of B cell growth and differentiation factors, including IL-5, to treat malignantly transformed cells in animals and man. Cells susceptible to treatment by the methods are those which have (or can be induced to have) receptors for the growth and differentiation factors, primarily leukemic cells. The methods described are said to act by producing terminally differentiated cells which do not further divide, not by causing cytotoxic effects that kill the cells.

## SUMMARY OF THE INVENTION

The present invention provides methods for treating solid tumors lacking IL-5 receptors in mammals.

More particularly, this invention provides methods for treating solid tumors in a mammal which comprise administering to a mammal afflicted with a solid tumor lacking IL-5 receptors an effective amount of IL-5 to inhibit the growth of the tumor.

## BRIEF DESCRIPTION OF THE FIGURES

This invention can be more readily understood by reference to the accompanying figures, in which:

Fig. 1 is Scatchard plot of the binding of $^{125}$I-IL-5 to KHOS cells (open squares) and to HL-60 cells (closed triangles).

Fig. 2 is a graphical representation of the inhibition of the growth of KHOS human osteogenic sarcoma cells in athymic mice by varying levels of recombinant human IL-5. Tumor volume is shown as a function of time, for IL-5 doses of 0 (open squares), 25 (closed diamonds), 100 (closed squares) and 400 (closed circles) μg/kg/day.

Fig. 3 is a graphical representation of the induction of membrane permeability in human retinal endothelial to the passage of $^{125}$I-BSA by IL-2 and IL-5. Percent maximal transfer of the labeled protein through the cellular membranes is shown as a function of time.

## DESCRIPTION OF THE INVENTION

All references cited herein are hereby incorporated in their entirety by reference.

The term "solid tumor" is defined herein as a non-hematopoietic carcinoma or sarcoma of epithelial, connective tissue, nervous tissue, thymic, ovarian, testicular, melanocytic or multiple tissue origin, the growth of which is inhibited in response to treatment with an effective amount of IL-5. Such tumors are characterized by an absence of specific IL-5 receptors on the surfaces of the cells comprising the tumors.

The term "lacking IL-5 receptors" is defined herein to mean that cells from a tumor in an $^{125}$I-IL-5 binding assay in the presence of saturating amounts of unlabeled IL-5 display essentially no specific IL-5 binding. The term "essentially no specific IL-5 binding" as applied to a given tumor means that the tumor has fewer than about 25 binding sites per cell, by Scatchard analysis.

Although a human osteogenic sarcoma is used for the purpose of illustration in the Example below, it must be understood that many other solid tumors will be encompassed by this invention. Whether a given solid tumor lacks IL-5 receptors and is thus susceptible to treatment by the methods of the invention can readily be determined from analysis of a biopsy specimen using labeled IL-5 in a receptor binding assay, e.g., as described below.

The lack of IL-5 receptors on tumors susceptible to treatment by the methods of this invention is also evidenced by a lack of direct effects by IL-5 on such cells *in vitro*. For example, recombinant human IL-5 had no direct effect on the proliferation of the cultured human osteogenic sarcoma KHOS cells described below.

As used herein, the term "treating solid tumors" means producing an anti-tumor response by the administration of IL-5 in which growth of a solid tumor is inhibited and/or regression of the tumor occurs.

Although it is presently believed that IL-5 acts to produce an eosinophil-mediated cytotoxic effect on the tumor cells, whether this possible mechanism is correct or not is not essential to the invention. In any event, the methods of this invention are to be distinguished from the induction of differentiation and consequential impairment of growth of tumor cells bearing IL-5 receptors which have been described in the prior art (International Patent Application Publication No. WO 88/06891).

IL-5 from any source can be used in this invention, e.g., from commercial or natural sources or through the application of chemical synthetic methods or recombinant DNA technology. "Recombinant IL-5 " is defined herein to mean IL-5 produced by expression of recombinant DNA (genomic or cDNA) encoding the same in a prokaryotic or eukaryotic expression system. The IL-5 used may be glycosylated or unglycosylated.

IL-5 can be prepared using known methods. For example, murine IL-5 has been isolated from medium conditioned by cultured helper T cells [McKenzie *et al.*, J. Immunol. *139*:2661 (1987)]. Preferably, however, IL-5 is made by the application of recombinant DNA techniques.

Since the nucleotide sequences of DNA encoding murine and human IL-5 are known [see e.g., Azuma *et al.*, Nucleic Acids Res. *14*:9149 (1986)], such DNAs can be chemically synthesized using the phosphoramidite solid support method of Matteucci *et al.* [J. Am. Chem. Soc. *103*:3185 (1981)], the method of Yoo *et al.* [J. Biol. Chem. *764*:17078 (1989)], or other well known methods. This can be done, for example, by synthesizing relatively small oligonucleotides and ligating them together, analogous to the way that Barr *et al.* (International Patent Application Publication No. WO 85/02200) chemically synthesized DNA encoding IL-2.

For example, a cell line capable of making IL-5 can be stimulated to make IL-5 mRNA, which can serve as a template to make IL-5 cDNA by standard methods. A cDNA library can then be constructed in which IL-5 cDNA can be identified using oligonucleotide probe mixtures based on the known sequence information. This cDNA can then be cloned and expressed in one of the many available bacterial, yeast or mammalian expression systems. This method has been used to produce recombinant rat, murine or human IL-5 [see, e.g., Tavernier *et al.*, DNA *8*:491 (1989); Minamitake *et al.*, J. Biochem. *107*:292 (1990); Uberla *et al.*, Cytokine *3*:72 (1991)]. Human IL-5 was produced in a commonly-owned U.S. patent application (Serial No. 07/615,061, filed November 16, 1990) by expressing cDNA encoding human IL-5 in Chinese hamster ovary (CHO) cells. Tsujimoto *et al.* [J. Biochem. *106*:23 (1989)] have also described the production of recombinant human IL-5 in CHO cells.

Alternatively, oligonucleotide probe mixtures based on known IL-5 nucleotide sequences can be used to identify IL-5 genes in genomic DNA libraries prepared by standard methods. DNA thus identified can be excised from the library by restriction endonuclease cleavage, sequenced and expressed in a eukaryotic expression system or (following intron deletion by standard methods if necessary) in a prokaryotic expression system. In this way, Campbell *et al.* [Proc. Natl. Acad. Sci. USA *84*:6629 (1987)] produced human IL-5 in monkey kidney (COS) cells.

Of course, both cDNA and genomic DNA libraries can be screened by the application of standard expression cloning methods, instead of by the use of oligonucleotide probes. IL-5 thus produced is detected through the use of known immunochemical or bioassay methods.

Finally, IL-5 can be purchased commercially, e.g., from Genzyme Corporation, Boston, MA.

The IL-5 used will preferably be that of the mammalian species being treated (e.g., human IL-5 is preferred for treating human beings). It is also preferred that glycosylated IL-5 be used (e.g., recombinant IL-5 produced in a eukaryotic expression system).

In accordance with the present invention, mammals are administered an effective amount of IL-5 to accomplish the above-described results. A dose of from about 1 to about 400 micrograms of IL-5 per kilogram of body weight per day is preferably administered. More preferably, mammals are administered a dose of about 1 to about 5 micrograms of IL-5 per kilogram of body weight per day.

The actual amount, frequency and period of administration of IL-5 will vary depending upon factors including but not limited to the tumor burden and tumor location, and the immunological status, nutritional state, general condition and age of the patient. Usually, the administration of the IL-5 will initially be daily and may be continued during the patient's lifetime if required. Dosages are usually initiated at a relatively low level and then gradually increased to a point at which unacceptable toxicity is observed as evidenced by, e.g., edema, weight loss, nausea, fever and vomiting. In the event that such toxicity is noted, treatment may be continued at a lower dosage level or after a recovery period during which the foregoing monitored parameters return to normal.

Administration of the dose can be intra-lesional, although parenteral administration by intraperitoneal, intravenous, subcutaneous or intramuscular injection or infusion or by any other acceptable systemic method is preferred. Presently, the subcutaneous or intramuscular route of administration is especially preferred. Continuous infusion can be carried out by mechanical pump or gravity feed.

Parenteral preparations that can be used include sterile solutions or suspensions. These preparations can be prepared with conventional pharmaceutically acceptable excipients and additives such as stabilizers and carriers. The solutions to be administered may be reconstituted lyophilized powders which may additionally contain, e.g., preservatives, buffers and dispersants.

The effect of IL-5 on the solid tumors of an afflicted mammal can be determined, e.g., by measuring a reduction of tumor volume (e.g., using calipers) and/or changes in tumor weight or in eosinophils, heterophils or mast cells.

## EXAMPLE

The present invention can be illustrated by the following, non-limiting Example.

## Materials

Human osteogenic sarcoma KHOS cells (ATCC CRL 1544) were obtained from the American Type Culture Collection, Rockville, MD. Ten-week old C57B1/6 mice and Nu/Nu Balb/c (athymic) mice were supplied by Charles River. Most tissue culture reagents and supplies were from JRH Biosciences. Fetal bovine serum (FBS) was from Hyclone Laboratories, Inc. Polycarbonate membrane transwell cups (8 micron pore size) were purchased from Costar Corporation, Cambridge, MA.

Hematoxylin and eosin (H&E) and Giemsa stains were obtained from Fisher Scientific Co. $^{125}$I-BSA (specific radioactivity 1-5 $\mu$Ci/$\mu$g) was a product of NEN DuPont.

Recombinant human IL-5 was produced by and purified from transformed cultured Chinese hamster ovary (CHO) cells, using standard methods as described in commonly owned U. S. application Serial No. 07/615,061, filed November 16, 1990. The amino acid sequence of the recombinant human IL-5 thereby obtained was the same as that described by Yokota *et al.* [Proc. Natl. Acad. Sci. USA *84*:7388 (1987)]. Recombinant human IL-2 was obtained from Genzyme Corporation.

## Cell Culture

KHOS cells used for inoculation were maintained in a 37°C, 5% $CO_2$ incubator in Eagle's Minimum Essential Medium (EMEM) supplemented with 10% FBS, 4 mM L-glutamine, 0.2% sodium bicarbonate, 50 U/ml penicillin and 50 $\mu$g/ml streptomycin. The cells were routinely passaged twice weekly at 1:20 split ratios into Bellco 75 $cm^2$ tissue culture flasks. Several days prior to use, the cells were passaged in Falcon 150 $cm^2$ tissue culture flasks at 1:10 split ratios.

## IL-5 Receptor Analysis

### Preparation of $^{125}$I-IL-5

Recombinant human IL-5 prepared from CHO cells was labeled with iodine-125 by the iodogen method and purified by DuPont NEN (Boston, MA) to a specific radioactivity of 160 μCi/μg. The $^{125}$IL-5 migrated in sodium dodecyl sulfate polyacrylamide gel electrophoresis [Laemmli, Nature 227:680 (1970)] under non-reducing conditions with an apparent molecular weight of about 40,000 daltons. This is the molecular weight expected for the dimeric form of IL-5, which McKenzie et al. [Mol. Immunol. 28:155 (1991)] have shown is essential for biological activity. When tested in the TF-I cell proliferation assay essentially as described by Kitamura et al. [J. Cell. Physiol. 140:323 (1989)], the $^{125}$I-IL-5 was found to have retained more than half of the biological activity of the unlabeled protein.

### Tumor Cell Preparation

KHOS cells used for binding studies were cultured as described above, harvested at log phase and treated with ethylenediaminetetraacetic acid (EDTA; 4 mM in calcium and magnesium-free PBS) to detach the cells from the culture dishes and washed twice with binding buffer (DMEM with 0.02% sodium azide). The cells were resuspended in binding buffer and equilibrated at 4°C prior to use.

The human promyelocytic leukemia cell line HL-60 (ATCC CCL 240), known to possess IL-5 receptors [Ingley et al., Blood 78:339 (1991)], was used as a positive control. The cells were maintained in Iscove's medium supplemented with 10% FBS and 4 mM L-glutamine. The cells were grown under alkaline conditions essentially as described by Fischkoff et al. [Cancer Res. 45:2065 (1985)] for 3 days, after which butyric acid was added to a final concentration of 0.4 mM. Although HL-60 cells are anchorage dependent, they too were treated with EDTA prior to use, so that the treatments of the KHOS and HL-60 cells were as similar as practicable.

To determine whether the cells possessed IL-5 receptors, binding experiments were performed at 4°C for 2 hours with $1 \times 10^6$ KHOS or HL-60 cells in 200 μl of binding buffer containing from $0.2 \times 10^6$ to $5.0 \times 10^6$ cpm $^{125}$I-IL-5, with or without a 1,000-fold excess of unlabeled IL-5. The tubes were gently rocked during the incubation, after which the cells were pelleted by centrifugation and resuspended in 150 μl of ice cold binding buffer. These suspensions were layered over 150 μl volumes of an oil mixture (1.1:1 dibutyl phthalate oil:dioctyl phthalate oil) and centrifuged at about 14,000 x g for 45 seconds to separate unbound $^{125}$I-IL-5 from the labeled cells.

The tubes were then dipped into liquid nitrogen to quick freeze the contents and the tips of the tubes were cut off and placed into polystyrene tubes and counted in an LKB Model 1272 gamma counter. Specific IL-5 binding was defined as the difference between total binding (in the absence of unlabeled IL-5) and non-specific binding in the presence of the 1,000-fold excess of unlabeled IL-5.

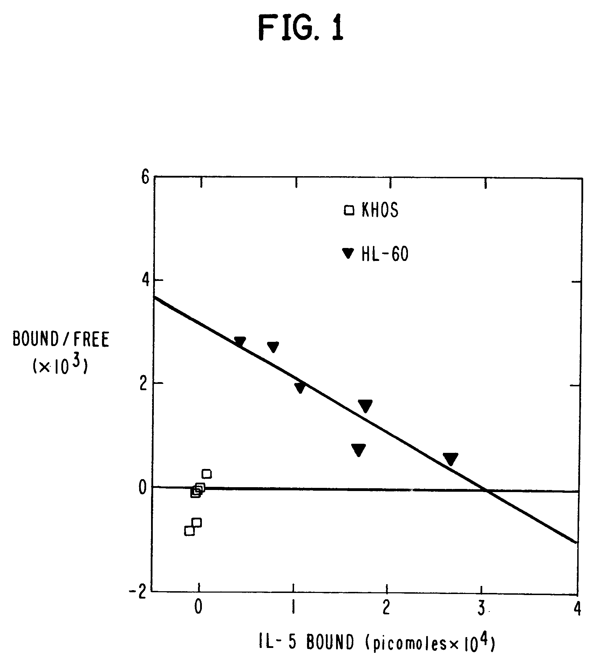

A Scatchard plot [Scatchard, Ann. N.Y. Acad. Sci. 51:660 (1949)] of the results is shown in Fig. 1, where it can be seen that under the conditions described above, the specific binding of $^{125}$I-IL-5 to the KHOS cells was essentially not detectable. The lower limit of detection was about 25 binding sites per cell. In contrast, dose-dependent specific binding of the $^{125}$I-IL-5 to the HL-60 cells was observed, showing that those cells possessed specific IL-5 receptors.

### Tumor Inhibition

A human tumor xenograft/athymic mouse system was used to illustrate the method of the present invention. In the absence of unexpected toxicity in human beings, results obtained in such systems are a good predictor of human clinical efficacy (Kelly et al., Cancer Surveys 8:741 (1989)] and thus useful for the preclinical screening of cytokines.

Groups of five of the above-mentioned athymic mice were injected subcutaneously with $2 \times 10^6$ KHOS cells in a volume of 0.2 ml of phosphate buffered saline (PBS). Beginning shortly thereafter on the date of tumor cell inoculation and continuing on a daily basis, the animals received intraperitoneal injections of control injection medium or 25, 100 or 400 μg per kilogram body weight recombinant human IL-5 in 0.2 ml of PBS.

At various times after tumor cell inoculation, three-dimensional caliper measurements were made to determine tumor volumes. After a total of 4 weeks, the animals were sacrificed and examined for tumor volume and tumor weight.

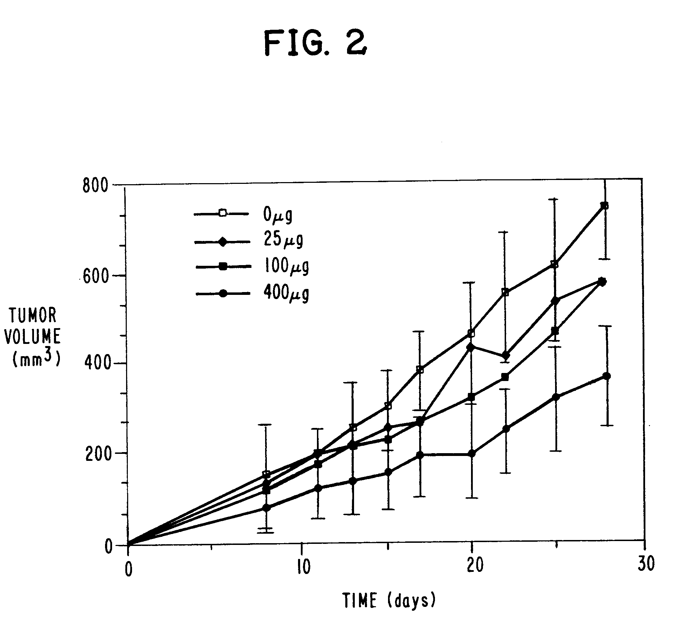

The effect of the IL-5 on tumor volume is shown in Fig. 2, where it can be seen that there was a dose-dependent decrease in tumor volumes in the groups treated with IL-5, compared to the control group. This is most evident at 20 days, where the curves from top to bottom represent 0, 25, 100 and 400 μg IL-5, respectively.

The results after 4 weeks of IL-5 treatment are shown for the same groups of animals in Table 1. Mean values of tumor size are shown in Table 1 in terms of tumor weight and volume, both as an absolute value and as a percentage of control (no IL-5) tumor volume. Values shown in parentheses are standard deviations. Values denoted by superscript letters were shown to be statistically significant by the Student's t test (Introduction to Probability and Statistics, Student's t Distribution. Small Sample Methods, H.L. Alder and E.B. Roessler, W.H. Freeman & Co., San Francisco, CA, pp. 153-173).

## Table 1

### Inhibition of Tumor Growth by IL-5

| IL-5 Dose (μg/kg/day) | Tumor Weight (grams) | Tumor Vol. (mm$^3$) | Tumor Vol. (% Control) |
|---|---|---|---|
| 0 | 0.5056 (0.034) | 726 (211) | 100.0 |
| 25 | 0.3609[a] (0.135) | 566 (155) | 78.0 |
| 100 | 0.3418[a] (0.106) | 564 (110) | 77.7 |
| 400 | 0.3074[b] (0.148) | 352[c] (110) | 48.5 |

[a] $p<0.10$
[b] $p<0.05$
[c] $p<0.02$

To further explore the pathological manifestations of recombinant human IL-5 administration, data were obtained from 8 athymic mice inoculated with KHOS cells as described above. Three of the animals were controls, which received only daily injections of inoculation medium. Five of the mice received daily intraperitoneal injections of 100 μg/kg recombinant human IL-5 as described above.

After four weeks from tumor cell inoculation, the animals were sacrificed. Sections (6 μm) from formalin-fixed, paraffin-embedded tissues were stained with H&E and Giemsa stains. Overall histologic evaluations were made on H&E-stained sections for KHOS neoplasm (subcutaneous), liver, kidney, pancreas, spleen, lung and bone marrow (femur and sternum). Eosinophil, heterophil and mast cell counts were made in both the tumors and perineoplastic stroma in tumor-containing skin sections The results for the evaluation of eosinophils, heterophils and mast cells are summarized in Tables 2-4, respectively.

## Table 2

### Eosinophil Evaluation

| Mouse Number | Perineoplastic[a] Stroma | Peripheral[b] Neoplasm | Central[b] Neoplasm |
|---|---|---|---|
| Control | | | |
| 1 | 7 | 2 | 0 |
| 2 | 10 | 4 | 0 |
| 3 | 19 | 2 | 0 |
| Average | 12 | 2.67 | 0 |
| Range | 7-19 | 2-4 | 0 |
| IL-5 | | | |
| 1 | 82 | 11 | 0 |
| 2 | 91 | 9 | 7 |
| 3 | 47 | 4 | 0 |
| 4 | 72 | 47 | 1 |
| 5 | 77 | 6 | 0 |
| Average | 73.8 | 15.4 | 1.6 |
| Range | 47-91 | 4-47 | 0-7 |

a   Total cell counts from 5 randomly-selected 63X fields
b   Total cell counts from 10 randomly-selected 63X fields

## Table 3

### Heterophil Evaluation

| Mouse Number | Perineoplastic[a] Stroma | Peripheral[b] Neoplasm | Central[b] Neoplasm |
|---|---|---|---|
| Control | | | |
| 1 | 10 | 8 | 0 |
| 2 | 16 | 7 | 1 |
| 3 | 6 | 6 | 0 |
| Average | 10.67 | 7 | 0.33 |
| Range | 6-16 | 6-8 | 0-1 |
| IL-5 | | | |
| 1 | 9 | 2 | 0 |
| 2 | 9 | 3 | 9 |
| 3 | 16 | 2 | 0 |
| 4 | 22 | 10 | 0 |
| 5 | 11 | 0 | 0 |
| Average | 13.4 | 3.4 | 1.8 |
| Range | 9-22 | 0-10 | 0-9 |

[a] Total cell counts from 5 randomly-selected 63X fields
[b] Total cell counts from 10 randomly-selected 63X fields

## Table 4

### Mast Cell Evaluation

| Mouse Number | Perineoplastic[a] Stroma | Peripheral[b] Neoplasm | Central[b] Neoplasm |
|---|---|---|---|
| Control | | | |
| 1 | 12 | 3 | 0 |
| 2 | 11 | 1 | 0 |
| 3 | 4 | 0 | 0 |
| Average | 9 | 1.33 | 0 |
| Range | 4-12 | 0-3 | 0 |
| IL-5 | | | |
| 1 | 6 | 0 | 0 |
| 2 | 7 | 0 | 9 |
| 3 | 11 | 0 | 0 |
| 4 | 13 | 1 | 0 |
| 5 | 10 | 1 | 0 |
| Average | 9.4 | 0.4 | 0 |
| Range | 6-13 | 0-1 | 0 |

[a]  Total cell counts from 5 randomly-selected 63X fields
[b]  Total cell counts from 10 randomly-selected 63X fields

The histologic evaluation revealed that all neoplasms from the treated and control mice had large central areas of coagulative and liquifactive necrosis which averaged about 40% of the mass. Control mice had minimal infiltration of leukocytes into the perineoplastic stroma and into the peripheral neoplasm. IL-5-treated mice had several to many leukocytes in the perineoplastic stroma and at the neoplasm-connective tissue stroma junction. Eosinophils were a prominent component in these infiltrates, but they were accompanied by heterophils and mononuclear cells (probably macrophages, lymphocytes and fibroblasts).

IL-5-related changes were not detectable in liver, kidney, pancreas, spleen and lung. One KHOS metastatic focus was seen in lung from a control mouse. Examination of bone marrow from femur and sternum suggested that increased eosinopoiesis had occurred.

In sum, intraperitoneal IL-5 treatment caused KHOS tumor growth inhibition, as measured by tumor weight and diameter at necropsy. This inhibition was associated with increased leukocytic infiltration in the perineoplastic stroma and at the neoplasm-stromal interface. Eosinophils were a prominent component in these infiltrates, suggesting that they may have been integral in affecting tumor growth.

### Vascular Leak Syndrome Analysis

As noted above, Kelly et al. [Cancer Surveys 8:741 (1989)] have stated that, in the absence of unexpected toxicity in human beings, the human tumor xenograft/athymic mouse system is a good predictor of the human clinical efficacy of cytokines. According to Kelly *et al.*, observations made in the system using tumor necrosis factor have been borne out in subsequent human clinical trials. Results obtained in human beings using IL-2, however, have not been as encouraging as might have been predicted from experimental animal studies.

This discrepancy in the case of IL-2 is believed to be due to a condition known as vascular leak syndrome, which in the treatment of human beings produces systemic toxicity. Vascular leak syndrome is thought to be due to increased capillary permeability, which results in a leakage of water and osmotically active proteinaceous components of the plasma [Puri *et al.*, Can. Immunol. Immunother. *28*:267 (1989)].

To determine whether IL-5 causes an increase in capillary endothelial cell permeability, the effect of IL-5 on the permeability of human retinal endothelial (HRE) cells to the passage of albumin was determined *in vitro*. This was accomplished by growing HRE cells in Dulbecco's Modified Eagle's (DME) medium with low glucose, 10% FBS, L-glutamine, penicillin and streptomycin past confluence on polycarbonate membranes (8 μm pore size) mounted in transwell cups fitted into the wells of 24-well tissue culture plates.

The HRE cells were then treated for various periods of time with human recombinant IL-2 ($10^4$ units/ml) or IL-5 ($10^5$ units/ml) in the above-mentioned DME medium. $^{125}$I-BSA ($2 \times 10^5$ cpm) was added to the cups, and diffusion of the BSA through the cell monolayers was measured after 90 minutes by counting aliquots of the liquid beneath the cups in a gamma counter. The results were calculated as follows:

$$\% \text{ transfer} = \frac{\text{total counts} - \text{ST}}{\text{total transfer} - \text{ST}} \times 100,$$

wherein ST (spontaneous transfer) is the average cpm from wells containing untreated cells, and total transfer is the average cpm from wells containing no cells.

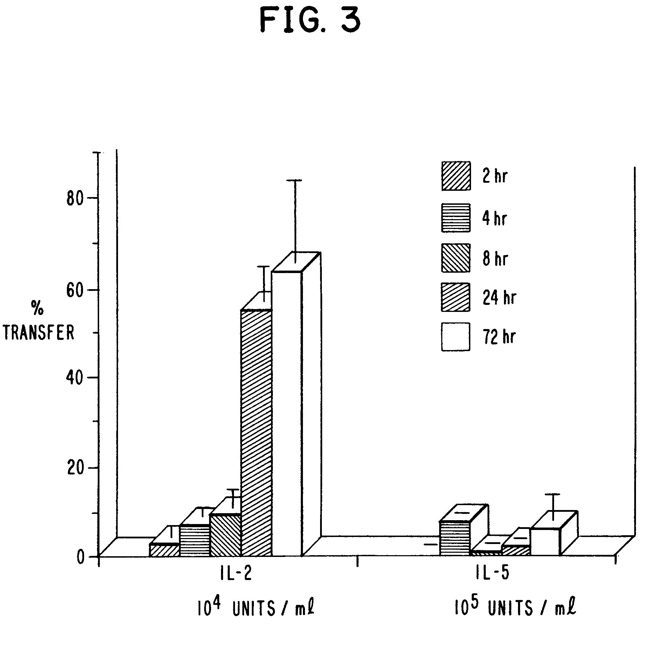

The results are shown in Fig. 3, where it can be seen that recombinant human IL-2 at a concentration of $10^4$ units/ml produced a marked increase in HRE cell permeability. In contrast, recombinant human IL-5 at a concentration an order of magnitude higher caused no significant increase in permeability. These data suggest that human IL-5 would not be expected to directly cause vascular leak syndrome in human patients.

Many modifications and variations of this invention can be made without departing from its spirit and scope, as will become apparent to those skilled in the art. The specific embodiments described herein are offered by way of example only, and the invention is to be limited only by the terms of t.he appended claims.

## Claims

1. A method for treating solid tumors in a mammal comprising administering to a mammal afflicted with a solid tumor lacking IL-5 receptors an effective amount of IL-5 to inhibit the growth of the tumor.

2. The method of claim 1 in which the IL-5 is recombinant human IL-5.

3. The use of IL-5 for treating solid tumors which lack IL-5 receptors.

4. The use of IL-5 for the manufacture of a medicament for treating solid tumors which lack IL-5 receptors.

5. The use of IL-5 as claimed in either claim 3 or 4 in which the IL-5 is recombinant human IL-5.

6. A pharmaceutical composition for treating solid tumors which lack IL-5 receptors comprising IL-5 and a pharmaceutically acceptable carrier.

7. The pharmaceutical composition of claim 6 in which the IL-5 is recombinant human IL-5.

# FIG. 1

# FIG. 2

# FIG. 3

## PARTIAL EUROPEAN SEARCH REPORT

European Patent
Office

which under Rule 45 of the European Patent Convention
shall be considered, for the purposes of subsequent
proceedings, as the European search report

Application Number

EP    92 30 8325
Page 1

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,Y | THE JOURNAL OF EXPERIMENTAL MEDICINE vol. 167, no. 1, 1 January 1988, NEW YORK;US. page 219224 A. F. LOPEZ ET AL 'Recombinant human interleukin 5 is a selective activator of human eosinophil function' * the whole document especially pages 222-223 * | 1-7 | A61K37/02 |
| Y | JOURNAL OF CLINICAL PATHOLOGY vol. 34, 1981, pages 1343 - 1348 D. LOWF ET AL 'Tumor-associated eosinophilia : a review' * the whole document * <br>---<br> -/-- | 1-7 | |

TECHNICAL FIELDS
SEARCHED (Int. Cl. 5)

A61K
C07K
C12N
C12P

### INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 15 DECEMBER 1992 | LE CORNEC N.D.R. |

**European Patent Office**

## PARTIAL EUROPEAN SEARCH REPORT

Application Number

EP 92 30 8325
Page 2

| | DOCUMENTS CONSIDERED TO BE RELEVANT | | CLASSIFICATION OF THE APPLICATION (Int. Cl. 5) |
|---|---|---|---|
| Category | Citation of document with indication, where appropriate, of relevant passages | Relevant to claim | |
| Y | CANCER<br>vol. 58, no. 6, 15 September 1986,<br>pages 1321 - 1327<br>KAZUNORI IWASAKI ET AL 'Malignant tumor<br>and eosinophils I. prognostic significance<br>in gastric cancer'<br>* the whole document *<br>--- | 1-7 | |
| Y | NEOPLASMA<br>vol. 31, no. 3, 1984,<br>pages 323 - 326<br>A. PASTRŇÁK ET AL 'Local eosinophilia in<br>stroma of tumors related to prognosis'<br>* the whole document *<br>--- | 1-7 | |
| D,A | WO-A-8 806 891 (AKTIEBOLAGET ASTRA)<br>* the whole document *<br>--- | 1-7 | |
| P,X | BLOOD<br>vol. 79, no. 12, 15 June 1992, NEW YORK;<br>US.<br>pages 3101 - 3109<br>C. J. SANDERSON 'Interleukin-5 ,<br>eosinophils , and disease'<br>* the whole article especially page 3105 *<br>----- | 1-7 | |

TECHNICAL FIELDS
SEARCHED (Int. Cl. 5)

EPO FORM 1503 03.82 (P04E10)

15

Sheet C                                    EP 92308325

Remark:

Although claims 1-3 and 5 partially are directed to a method of
treatment of the human/animal body, the search has been carried
out and based on the alleged effects of the composition/compound.